(19) Europäisches Patentamt
European Patent Office
Office européen des brevets

(11) EP 2 039 395 A2

# (12) DEMANDE DE BREVET EUROPEEN

(43) Date de publication:
**25.03.2009 Bulletin 2009/13**

(51) Int Cl.:
***A61Q 1/10*** *(2006.01)* ***A61K 8/92*** *(2006.01)*
***A61K 8/97*** *(2006.01)*

(21) Numéro de dépôt: **08161857.1**

(22) Date de dépôt: **05.08.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **21.08.2007 FR 0757101**

(71) Demandeur: **L'Oréal**
**75008 Paris (FR)**

(72) Inventeur: **Arditty, Stéphane**
**91160 BALLAINVILLIERS (FR)**

(74) Mandataire: **Boulard, Denis**
**L'Oréal**
**River Plaza - DIPI**
**25-29 Quai Aulagnier**
**92665 Asnières-sur-Seine (FR)**

## (54) Composition cosmétique comprenant un dérivé d'huile de soja et/ou un de ses sels

(57) La présente demande concerne une composition cosmétique de revêtement des cils comprenant une phase aqueuse et un système émulsionnant comprenant au moins un dérivé d'huile de soja et/ou un de ses sels.

EP 2 039 395 A2

## Description

La présente demande se rapporte au domaine du maquillage ou du soin des cils ou mascaras.
Les compositions de revêtement des cils tels que les mascaras sont généralement des compositions de maquillage, des compositions à appliquer sur un maquillage (encore appelées « top-coat »), ou encore des compositions de soin cosmétique des cils.
Les mascaras sont notamment préparés selon deux types de formulation : les mascaras aqueux dits mascaras crèmes, sous forme de dispersion de cires dans l'eau ; les mascaras anhydres ou à faible teneur en eau, dits mascaras waterproofs, sous forme de dispersions de cires dans des solvants organiques.
La présente demande concerne plus spécifiquement les mascaras aqueux.
L'application de mascara permet d'augmenter le volume des cils et en conséquence d'augmenter l'intensité du regard. Pour cela, il existe de nombreux mascaras épaississants ou volumateurs dont le principe consiste à déposer le maximum de matière sur les cils de manière à obtenir un effet volumateur (ou chargeant).

C'est en particulier à travers la quantité de particules solides (notamment les cires, qui permettent de structurer la composition), que peuvent être ajustées les spécificités d'application recherchées pour les compositions, comme par exemple leur fluidité ou consistance, ainsi que leur pouvoir épaississant (encore appelé pouvoir chargeant ou maquillant). Ces particules solides sont dispersées dans le mascara crème à l'aide d'un système tensioactif.
Parmi les émulsionnants ou systèmes émulsionnant classiques, on compte notamment les systèmes émulsionnants à base de stéarate de triéthanolamine.

Le problème posé dans la présente demande est de proposer un mascara dans lequel non seulement les cires mais aussi les pigments sont dispersés de façon homogène, ledit mascara présentant une texture suffisamment épaisse pour obtenir un dépôt chargeant, volumateur sur les cils, et présentant une consistance satisfaisante permettant une application facile sur les cils et un dépôt lisse et homogène.

De manière surprenante et inattendue, les inventeurs de la présente demande ont résolu ce problème au moyen d'un système émulsionnant comprenant au moins un dérivé d'huile de soja et/ou l'un de ses sels.

Les inventeurs de la présente demande ont pu observer que le système émulsionnant à base de dérivés du soja défini dans la présente demande permet une bonne dispersion des pigments et/ou des cires, cette dispersion est de la qualité de celles obtenues avec les systèmes émulsionnants à base de stéarate de triéthanolamine. Cette composition permet d'obtenir un maquillage chargeant des cils et une consistance satisfaisante compatible avec l'obtention d'un dépôt lisse et homogène sur lesdites fibres.

Les compositions conformes à l'invention peuvent avoir un comportement viscoélastique.

De façon générale, un matériau est dit viscoélastique quand, sous l'effet du cisaillement, il possède à la fois les caractéristiques d'un matériau élastique, c'est à dire capable de stocker de l'énergie et les caractéristiques d'un matériau visqueux, c'est à dire capable de dissiper de l'énergie.

Le comportement viscoélastique des compositions conformes à l'invention peut être plus particulièrement caractérisé par son module de rigidité G. Ce paramètre est notamment défini dans l'ouvrage "Initiation à la rhéologie", G. Couarraze et J.L. Grossiord, 2$^{ème}$ édition, 1991, Edition Lavoisier-Tec 1 Doc.

Les mesures sont effectuées sur un rhéomètre à contrainte imposée, RS 600 de la société ThermoRhéo, équipé d'un bain thermostaté et d'un mobile en acier inoxydable à géométrie cône/plan, de diamètre 35 mm et d'angle 2°. Les 2 surfaces sont « sablées » pour limiter les phénomènes de glissement aux parois.

Les mesures sont effectuées à 25°C $\pm$ 1 °C.

Les mesures dynamiques sont réalisées en appliquant une variation harmonique de la contrainte. Dans ces expériences, les amplitudes de la contrainte de cisaillement (notée $\tau$) et de la déformation de cisaillement (notée $\gamma$) sont faibles de manière à rester dans les limites du domaine viscoélastique linéaire de la composition (conditions permettant d'évaluer les caractéristiques rhéologiques de la composition au repos).

Le domaine linéaire viscoélastique est généralement défini par le fait que la réponse du matériau (i.e. la déformation) est à tout moment directement proportionnelle à la valeur de la force appliquée (i.e. la contrainte). Dans ce domaine, les contraintes appliquées sont faibles et le matériau subit des déformations sans modifier sa structure microscopique. Dans ces conditions, le matériau est étudié « au repos » et de façon non destructive.

La composition est soumise à un cisaillement harmonique selon une contrainte $\tau(t)$ variant de façon sinusoïdale selon une pulsation $\omega$ ($\omega = 2\Pi\nu$), $\nu$ étant la fréquence du cisaillement appliqué. La composition ainsi cisaillée subie une contrainte $\tau(t)$ et répond selon une déformation $\gamma(t)$ correspondant à des micro déformations pour lesquelles le module de rigidité varie peu en fonction de la contrainte imposée.

La contrainte $\tau(t)$ et la déformation $\gamma(t)$ sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. L'élasticité $\delta$ est l'angle de déphasage entre la contrainte et la déformation.

Les mesures sont effectuées à une fréquence de 1 Hz (v= 1 Hz).

On applique des contraintes croissantes à l'échantillon en partant d'une contrainte initiale égale à 0,01 Pa pour arriver à une contrainte finale de 1000 Pa, les contraintes n'étant appliquées qu'une seule fois.

On mesure ainsi l'évolution du module de rigidité G (correspondant au rapport de $\tau o$ sur $\gamma o$) et de l'élasticité $\delta$ (correspondant à l'angle de déphasage de la contrainte appliquée par rapport à la déformation mesurée) en fonction de la contrainte $\tau(t)$ appliquée.

On mesure en particulier la déformation de la composition pour la zone de contrainte dans laquelle la variation du module de rigidité G et de l'élasticité $\delta$ est inférieure à 7 % (zone des microdéformations) et on détermine ainsi les paramètres dits « plateaux » Gp et $\delta$p.

La composition présente par exemple un module de rigidité plateau Gp supérieur ou égal à 10 Pa, préférentiellement supérieur ou égal à 50 Pa, pouvant aller jusqu'à $10^6$ Pa et mieux jusqu'à $5.10^5$Pa.

Un premier objet de la présente demande est une composition cosmétique de revêtement des cils comprenant une phase aqueuse et un système émulsionnant comprenant au moins un dérivé d'huile de soja et/ou un sel dudit dérivé.

Un autre objet de la présente demande est un procédé de maquillage ou de soin non thérapeutique des cils comprenant l'application sur lesdits cils de la composition selon la présente demande.

Un autre objet de la présente demande concerne les utilisations de la composition selon la présente demande, en particulier l'utilisation de cette composition pour obtenir un maquillage homogène et/ou volumateur des cils.

D'autres caractéristiques, propriétés et avantages de la présente invention apparaîtront plus clairement à la lecture de la description et des exemples qui suivent.

Système émulsionnant

Le système émulsionnant de la composition selon l'invention comprend au moins un dérivé d'huile de soja et/ou un sel dudit dérivé.

Par dérivé de l'huile de soja et leurs sels, on entend en particulier les acides gras et sels d'acides gras dérivés de l'huile de soja (dont le nom INCI est « glycine soja oil » ou « soybean oil »).

Le sel ou dérivé d'huile de soja peut être en particulier choisi parmi :

- les sels de métaux alcalins tels que Na, Li, K, de préférence Na ou K, et d'acides gras issus du soja, tels que le potassium soyate comme par exemple celui qui est commercialisé par la société Noveon,
- les esters d'acides gras dérivés de l'huile de soja et d'alcools, en particulier de diols tels que l'isopropanol comme le composé portant le nom INCI propylene glycol soyate tel que celui qui est commercialisé par A&E Connock,
- les dérivés oxypropylénés ou oxyéthylénés d'acides gras dérivés de l'huile de soja, comprenant en particulier de 5 à 10 unités oxyéthylénées ou oxypropylénées, tels que le PEG-9 soyate commercialisé sous la référence Silwax DMC-SOY par la société Siltech LLC,
- les esters de sucres et d'acides gras dérivés de l'huile de soja tels que le sucrose polysoyate commercialisé sous le nom SEFA Soyate par Procter & Gamble Chemicals,
- les dérivés obtenus par transestérification de l'huile de soja tels que le Soybean oil glycereth-8 esters commercialisé sous le nom Resplanta Soja par la société Res Pharma,
- et leurs mélanges.

Selon un mode de réalisation, le dérivé d'huile de soja et/ou son sel utilisé dans la composition selon l'invention n'est pas un alkyl soyate, tel que le méthyl soyate.

Selon l'invention, le dérivé d'huile de soja et/ou son sel utilisé dans la composition est différent de la lécithine de soja.

Selon un mode de réalisation, on utilise en particulier les sels de métaux alcalins tels que Na, Li, K, de préférence Na ou K, et d'acides gras issus du soja, tels que le potassium soyate.

Les dérivés d'huile de soja et leurs sels peuvent être présents dans la composition en une teneur allant de 0,1 à 15% en poids, de préférence de 0,5 à 12% en poids et mieux de 1 à 10% en poids par rapport au poids total de la composition.

De préférence les dérivés d'huile de soja et leurs sels constituent le système tensioactif principal de la composition.

Par « système tensioactif principal », on entend un système qui, en son absence, ne conduit pas à la formation d'une composition stable.

Par « stable », on entend une composition qui, après avoir été placée dans une étuve à 45°C pendant deux mois, ne présente pas, après retour à température ambiante, de grains perceptibles au toucher lorsqu'une couche fine

de la composition est cisaillée entre les doigts.

Avantageusement, les dérivés d'huile de soja et leurs sels constituent l'unique système tensioactif de la composition.

Par « unique » on entend que tout éventuel système tensioactif additionnel est présent en une teneur n'excédant pas 1%, et de préférence n'excédant pas 0,5%. De préférence encore, par « unique » on désigne une absence totale de tout autre système tensioactif.

La composition selon l'invention comprend bien entendu un milieu physiologiquement acceptable. Par « composé ou milieu physiologiquement acceptable » au sens de la présente demande, on entend un composé ou milieu dont l'utilisation est compatible avec une application sur les cils.

Phase aqueuse

La composition selon l'invention comprend une phase aqueuse, qui peut former la phase continue de la composition.

Par composition à phase continue aqueuse, on entend que la composition présente une conductivité, mesurée à 25 °C, supérieure ou égale à 23 $\mu$S/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse comprend de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$-$C_4$ et les aldéhydes en $C_2$-$C_4$.

La phase aqueuse (eau et éventuellement le solvant miscible à l'eau) est généralement présente dans la composition selon la présente demande en une teneur allant de 1 % à 95 % en poids, par rapport au poids total de la composition, de préférence allant de 3 % à 80 % en poids, et préférentiellement allant de 5 % à 60 % en poids.

Le système émulsionnant peut en outre contenir au moins un agent tensioactif additionnel choisi de manière appropriée pour l'obtention d'une émulsion cire dans eau ou huile-dans-eau.

En particulier, on peut utiliser un émulsionnant possédant à 25 °C une balance HLB (hydrophile-lipophile balance) au sens de GRIFFIN, supérieure ou égale à 8.

Ces agents tensioactifs additionnels peuvent être choisis parmi les agents tensioactifs non ioniques, anioniques, cationiques, amphotères ou encore les émulsionnants tensioactifs. On peut se reporter au document "Encyclopedia of Chemical Technology, KIRK-OTHMER", volume 22, p.333-432, 3ème édition, 1979, WILEY, pour la définition des propriétés et des fonctions (émulsionnant) des tensioactifs, en particulier p. 347-377 de cette référence, pour les tensioactifs anioniques, amphotères et non ioniques.

Ces tensioactifs additionnels peuvent être préférentiellement choisis parmi :

a) les agents tensioactifs non ioniques de HLB supérieur ou égal à 8 à 25 °C, utilisés seuls ou en mélange; on peut citer notamment :

- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 20 à 1000 groupes oxyéthylénés et/ou oxypropylénés) d'alcools gras (notamment d'alcoolen $C_8$-$C_{24}$, et de préférence en $C_{12}$-$C_{18}$) tels que l'éther oxyéthyléné de l'alcool cétéarylique à 30 groupes oxyéthylénés (nom CTFA "Ceteareth-30 "), l'éther oxyéthyléné de l'alcool stéarylique à 20 groupes oxyéthylénés (nom CTFA "Steareth-20 ") tel que le BRIJ 78 commercialisé par la société UNIQEMA, l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7® par SHELL CHEMICALS,
- les esters d'acide gras (notamment d'acide en $C_8$-$C_{24}$, et de préférence en $C_{16}$-$C_{22}$) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQEMA,

- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60® par la société UNIQUEMA,
- la diméthicone copolyol, telle que celle vendue sous la dénomination 02-5220® par la société DOW CORNING,
- la diméthicone copolyol benzoate (FINSOLV SLB 101® et 201® de la société FINTEX),
- les copolymères d'oxyde propylène et d'oxyde d'éthylène, également appelés polycondensats OE/OP,
- et leurs mélanges. Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.

b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :

- les esters et éthers d'oses tels que les stéarate de sucrose, cocoate de sucrose, stéarate de sorbitan et leurs mélanges comme l'Arlatone 2121® commercialisé par la société ICI ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- le mélange de cyclométhicone/diméthicone copolyol vendu sous la dénomination Q2-3225C® par la société DOW CORNING.
- c) Les tensioactifs anioniques tels que :
- les sels d'acides gras en C16-C30 notamment ceux dérivant des amines, comme le stéarate de triéthanolamine et/ou le stéarate d'amino-2-méthyl-2-propane diol-1,3 ; mais de préférence la composition selon la présente demande ne comprend moins 1% de stéarate de triéthanolamine ;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ou le phosphate de monocétyle monopotassique (Amphisol K de Givaudan) ;
- les iséthionates ;
- et leurs mélanges.

La composition conforme à l'invention peut également contenir, outre le dérivé d'huile de soja , un ou plusieurs tensioactifs amphotères comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

Dans la composition conforme à l'invention, la teneur totale en agents tensioactifs peut aller de 1 à 30 % en poids par rapport au poids total de la composition, de préférence de 1 à 20 % et mieux de 2 à 15 % en poids.

Selon un mode de réalisation, la composition cosmétique selon la présente demande comprend moins de 1%, de préférence moins de 0,5% en poids de triéthanolamine, et mieux, est exempte de triéthanolamine.

Selon une variante, la composition cosmétique selon la présente demande comprend moins de 1 %, de préférence moins de 0,5% en poids de stéarate de triéthanolamine, et mieux, est exempte de stéarate de triéthanolamine.

Selon un mode de réalisation, la composition comprend en outre un co-tensioactif choisi parmi les alcools gras, comprenant de préférence de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.

On utilise de préférence un alcool gras comprenant de 10 à 26 atomes de carbone, mieux de 10 à 24 atomes de carbone et encore mieux de 14 à 22 atomes de carbone.

On peut citer notamment comme alcools gras utilisables dans la composition les alcools laurique, myristique, cétylique, stéarylique, oléique, cétéarylique (mélange d'alcool cétylique et stéarylique), béhénique, érucique et leurs mélanges. On utilise de préférence l'alcool cétylique.

De tels alcools gras sont notamment commercialisés sous la dénomination NAFOL par la société SASOL.

L'alcool gras peut être présent en une teneur allant de 0,2 à 20% en poids, de préférence de 0,3 à 10% en poids par rapport au poids total de la composition.

Cire(s)

La composition selon la présente demande comprend avantageusement au moins une cire.

Par cire au sens de la présente invention, on entend un composé lipophile, solide à température ambiante (25 °C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

La cire peut être présente en une teneur allant de 0,1 à 50 % en poids par rapport au poids total de la composition, mieux de 1 à 40 % et encore mieux de 5 à 30% en poids.

On peut notamment utiliser les cires hydrocarbonées comme la cire d'abeille, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A-2792190.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s

à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II):

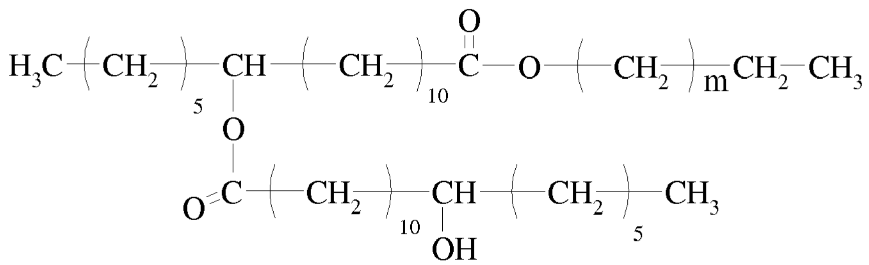

(II)

dans laquelle m est un entier allant de 18 à 38, ou un mélange de composés de formule (II).

Une telle cire est notamment vendue sous les dénominations "Kester Wax K 82 P®" et "Kester Wax K 80 P®" par la société KOSTER KEUNEN.

Les cires citées ci-dessus présentent généralement un point de fusion commençante inférieur à 45 °C.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 $\mu$m.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 $\mu$m (notamment allant de 0,02 $\mu$m à 0,99 $\mu$m), de préférence inférieures à 0,5 $\mu$m (notamment allant de 0,06 $\mu$m à 0,5 $\mu$m).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les compositions selon la présente demande peuvent aussi contenir au moins un polymère filmogène hydrophile ou lipophile.

Dans la présente demande, on entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonnée ou siliconnée.

De façon générale, la teneur en "polymère filmogène" des compositions selon la présente demande va de 0,1 à 40 %, de préférence de 0,5 à 30 % et mieux de 1 à 20 % en poids, par rapport au poids total de la composition,

Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques ,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate, et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Avalure UR-445® et Sancure 2060® par la société NOVEON, Impranil 85® par la société BAYER, Aquamere H-1511® par la société HYDROMER; les sulfopolyesters vendus sous le nom de marque Eastman AO® par la société Eastman Chemical Products, les dispersions vinyliques comme le Mexomère PAM®, les dispersions aqueuses de polyvinyl acétate comme le "Vinybran®" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur®" par la société AIR PRODUCTS ou "Duromer®" de NATIONAL STARCH, les dispersions type core/shell : par exemple celles commercialisées par la société ATOFINA sous la référence Kynar (core : fluoré-shell : acrylique) ou encore ceux décrits dans le document US 5 188 899 (core ; silice - shell : silicone) et leurs mélanges.

Le polymère lipophile peut être en solution ou en dispersion dans une phase solvant non aqueuse

Les compositions selon la présente demande peuvent aussi contenir au moins un gélifiant hydrophile, ils peuvent être choisi parmi :

- les homo- ou copolymères d'acides acrylique ou méthacrylique ou leurs sels et leurs esters et en particulier les produits vendus sous les dénominations VERSICOL F® ou VERSICOL K® par la société ALLIED COLLOID, ULTRAHOLD 8® par la société CIBA-GEIGY, les acides polyacryliques de type SYNTHALEN K,
- les copolymères d'acide acrylique et d'acrylamide vendus sous la forme de leur sel de sodium sous les dénominations

RETEN® par la société HERCULES, les sels de sodium d'acides polyhydroxycarboxyliques vendus sous la dénomination HYDAGEN F® par la société HENKEL,

- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL® ou SIMULGEL® commercialisés par la société SEPPIC, et
- les copolymères AMPS/méthacrylates d'alkyle polyoxyéthylénés (réticulés ou non), et leurs mélanges.
- les polyuréthanes associatifs tels que le polymère $C_{16}$-$OE_{120}$-$C_{16}$ de la société SERVO DELDEN (commercialisé sous le nom SER AD FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vensus sous forme pure) ou le DW 1206B de chez RHOM & HAAS à chaîne alkyle en $C_{20}$ et à liaison uréthane, vendu à 20 % en matière active dans l'eau. On peut aussi utiliser des solutions ou dispersions de ces polyuréthanes associatifs notamment dans l'eau ou en milieu hydroalcoolique. A titre d'exemple, de tels polymères on peut citer, le SER AD fx1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM& HAAS, ou bien encore le Borchigel LW 44 de la société BORCHERS,
- et leurs mélanges.

Certains polymères filmogènes hydrosolubles cités plus haut peuvent également jouer le rôle de gélifiant hydrosoluble.

Les gélifiants hydrophiles peuvent être présents dans les compositions selon l'invention en une teneur allant de 0,05 à 40% en poids par rapport au poids total de la composition, de préférence de 0,1 à 20% et mieux de 0,5 à 15% en poids.

Les compositions selon la présente demande peuvent aussi contenir au moins une ou plusieurs huiles ou solvant organique.

Par huile ou solvant organique, on entend un corps non aqueux liquide à température ambiante et pression atmosphérique. L'huile peut être volatile ou non volatile.

Par " huile ou solvant organique volatile", on entend au sens de l'invention tout milieu non aqueux susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par "huile non volatile", on entend une huile restant sur les matières kératiniques à température ambiante et pression atmosphérique au moins plusieurs heures et ayant notamment une pression de vapeur inférieure à $10^{-3}$ mm de Hg (0,13Pa).

L'huile peut être présente dans la composition dans une teneur allant de 0,05 à 30 %, de préférence 0,1 à 15 % en poids par rapport au poids total de la composition. La composition selon l'invention peut comprendre des huiles volatiles et/ou des huiles non volatiles, et leurs mélanges.

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars®" ou de "Permetyls®", les esters ramifiés en $C_8$-$C_{16}$, le néopentanoate d'isohexyle, et leurs mélanges. D'autres huiles hydrocarbonées volatiles comme les distillats de pétrole, notamment ceux vendus sous la dénomination "Shell Solt®" par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes ($6.10^{-6}$ $m^2/s$), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy

butane ou le perfluorométhylcyclopentane.

Chacune des compositions conformes à l'invention peut également comprendre au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools

ou de polyalcools comme le dioctanoate de propylène glycol ; les esters hydroxylés comme le lactate d'isostéaryle, le malate de di-isostéaryle ; et les esters du pentaérythritol;

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
  et leurs mélanges.

Les huiles de silicone non volatiles utilisables dans l'une ou l'autre des compositions (i) ou (ii) conformes à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans les compositions conformes à l'invention sont notamment des huiles fluorosiliconées, des polyéthers fluorés, des silicones fluorées telles que décrit dans le document EP-A-847752.

La teneur en huile ou solvant organique non volatile dans la composition conforme à l'invention va de 0,01 à 30 % en poids, en particulier de 0,1 à 25 % en poids, et mieux de 0,1 à 20 % par rapport au poids total de la composition.

De préférence, les compositions conformes à l'invention comprennent au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique

ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile

de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et

Expancel®007WU par la Société AKZO NOBEL.

Les charges peuvent représenter de 0,1 à 25 %, en particulier de 0,2 à 20 % en poids par rapport au poids total de la composition.

Les compositions conformes à l'invention peuvent également comprendre au moins une fibre qui permet une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Les compositions conformes à l'invention peuvent également comprendre au moins un actif cosmétique.

Comme actifs cosmétiques pouvant être utilisés dans les compositions conformes à l'invention, on peut citer notamment les antioxydants, les conservateurs, les parfums, les neutralisants, les émollients, les épaississants, les agents de coalescence, les plastifiants, les hydratants, les vitamines et les filtres en particulier solaires, et leurs mélanges.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

De préférence la composition selon l'invention est non rincée.

La composition selon l'invention peut être conditionnée dans un récipient délimitant au moins un compartiment

qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Les exemples suivants sont donnés à titre illustratif de la présente invention, et ne sauraient en limiter la portée.

Exemples

Les compositions suivantes ont été réalisées. Les quantités indiquées sont exprimées en pourcentage massique par rapport au poids total de la composition.

**Exemple 1 :**

- Cire d'abeille 10%
- Cire de candellila 10%
- Hydrogenated stearyl olive esters
  (Phytowax Olive 18L57 de chez Sophim) 10%
- Potassium soyate (de Noveon) 5%
- Potassium cocoate 0,5%
- Glycérine 0,58%
- Hydroxyéthylcellulose 0,9%
- Antimousse (siméthicone) 0,2%
- Oxyde de fer 7%
- Conservateurs qs
- Eau qsp 100

La phase grasse comprenant les cires est chauffée à 98°C puis on ajoute la phase aqueuse comprenant la glycérine, l'eau et les tensioactifs ainsi que les pigments préalablement broyés.

**Exemple 2 :**

- Cire d'abeille 10%
- Cire de candellila 10%
- Hydrogenated stearyl olive esters
  (Phytowax Olive 18L57 de chez Sophim) 10%
- Potassium soyate (de Noveon) 2,5%
- Potassium cocoate 0,25%
- Glycerin 0,29%
- Hydroxyéthylcellulose 0,9%

- Oxyde de fer 7%
- Antimousse (siméthicone) 0,2%
- Conservateurs qs
- eau qsp 100

Le module de rigidité G* de chacune des compositions est mesurée selon le protocole décrit plus haut.

| Résultats | **Exemple 1** | **Exemple 2** |
|---|---|---|
| G* (en Pa) | 1500 | 7000 |

Les mascaras des exemples 1 et 2 présentent une consistance satisfaisante et une bonne dispersion des cires et pigments qui assure une teinte noire, telle que recherchée pour ce type de produit.
Ces mascaras s'appliquent facilement sur les cils et forment un dépôt chargeant, lisse et homogène

**Revendications**

1. Composition cosmétique de revêtement des cils comprenant une phase aqueuse, au moins une matière colorante et un système émulsionnant **caractérisé en ce que** le système émulsionnant comprend au moins un dérivé d'huile de soja et/ou un sel dudit dérivé, ledit dérivé étant distinct du méthyl soyate et choisi parmi :

   - les sels de métaux alcalins et d'acides gras issus du soja,
   - les esters d'acides gras dérivés de l'huile de soja et d'alcools,
   - les dérivés oxypropylénés ou oxyéthylénés d'acides gras dérivés de l'huile de soja, comprenant en particulier de 5 à 10 unités oxyéthylénées

   ou oxypropylénées,

   - les esters de sucres et d'acides gras dérivés de l'huile de soja,
   - les dérivés obtenus par transestérification de l'huile de soja,
   - et leurs mélanges.

2. Composition selon la revendication 1, **caractérisée en ce que** le dérivé d'huile de soja ou son sel est choisi parmi le potassium soyate, le propylene glycol soyate, le PEG-9 soyate, le sucrose polysoyate, le Soybean oil glycereth-8 esters et leurs mélanges.

3. Composition selon l'une des revendications précédentes, **caractérisée en ce que** le dérivé d'huile de soja est le potassium soyate.

4. Composition selon l'une des revendications précédentes, **caractérisée en ce que** les dérivés d'huile de soja et leurs sels sont présents en une teneur allant de 0,1 à 15% en poids, de préférence de 0,5 à 12% en poids et mieux de 1 à 10% en poids par rapport au poids total de la composition.

5. Composition selon l'une quelconque des revendications précédentes, **caractérisée en ce que** les dérivés d'huile de soja ou leurs sels constituent le système tensioactif principal de la composition.

6. Composition selon l'une des revendications précédentes, **caractérisée en ce que** les dérivés d'huile de soja ou leurs sels constituent l'unique système tensioactif de la composition.

7. Composition cosmétique selon l'une des revendications précédentes, **caractérisée en ce qu'**elle comprend moins de 1%, de préférence moins de 0,5% en poids de triéthanolamine, et mieux, est exempte de triéthanolamine.

8. Composition cosmétique selon l'une des revendications précédentes, **caractérisée en ce qu'**elle comprend moins de 1%, de préférence moins de 0,5% de en poids de stéarate de triéthanolamine. et mieux, est exempte de stéarate de triéthanolamine.

**9.** Composition cosmétique selon l'une des revendications précédentes **caractérisée en ce que** la phase aqueuse est formée d'eau ou d'un mélange d'eau et d'au moins un solvant hydrosoluble.

**10.** Composition cosmétique selon l'une des revendications précédentes, **caractérisée en ce que** la phase aqueuse est présente en une teneur allant de 1 % à 95 % en poids, par rapport au poids total de la composition, de préférence allant de 3 % à 80 % en poids, et préférentiellement allant de 5 % à 60 % en poids.

**11.** Composition cosmétique selon l'une des revendications précédentes **caractérisée en ce qu'**elle comprend au moins une cire

**12.** Composition selon la revendication précédente, **caractérisée en ce que** la cire est présente en une teneur allant de 0, 1 % à 50% en poids, par rapport au poids total de la composition, de préférence de 1% à 40% en poids, et préférentiellement allant de 5% à 30% en poids.

**13.** Composition cosmétique selon l'une des revendications précédentes **caractérisée en ce qu'**elle comprend au moins un polymère filmogène hydrophile ou lipophile et/ou au moins un gélifiant hydrophile.

**14.** Composition cosmétique selon l'une des revendications précédentes **caractérisée en ce qu'**elle comprend au moins un additif choisi parmi les huiles, les charges, les fibres, les antioxydants, les conservateurs, les parfums, les neutralisants, les émollients, les épaississants, les agents de coalescence, les plastifiants, les hydratants, les vitamines et les filtres en particulier les filtres solaires, et leurs mélanges.

**15.** Procédé de maquillage ou de soin non thérapeutique des cils comprenant l'application sur lesdites cils de la composition selon l'une des revendications précédente.

**16.** Utilisation de la composition selon l'une des revendications 1 à 14 pour obtenir un maquillage homogène et/ou volumateur des cils

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2792190 A
- US 5188899 A
- EP 847752 A
- US 4887622 A
- FR 2796529
- FR 2761959
- FR 2792618

### Littérature non-brevet citée dans la description

- Encyclopedia of Chemical Technology, KIRK-OTHMER. WILEY, 1979, vol. 22, 333-432
- Microemulsions Theory and Practice. Academic Press, 1977, 21-32